# EUROPÄISCHE PATENTSCHRIFT

(11) **EP 4 369 981 B1**
(45) Veröffentlichungstag und Bekanntmachung des Hinweises auf die Patenterteilung: **26.03.2025**
(21) Anmeldenummer: 22747242.0
(22) Anmeldetag: 15.07.2022
(51) Int. Cl.: A44C 5/00, A44C 5/14, A44C 27/00, G04B 37/14

(54) **MOBILE ELEKTRONIKVORRICHTUNG**
MOBILE ELECTRONICS DEVICE
DISPOSITIF ÉLECTRONIQUE MOBILE

(30) Priorität: 16.07.2021 AT 1272021
(43) Veröffentlichungstag der Anmeldung: 22.05.2024
(73) Patentinhaber: Flasher GmbH, 8010 Graz (AT)
(72) Erfinder: RECH, Alexander, 8010 Graz (AT); WÖCKL, Ines, 8010 Graz (AT)
(74) Vertreter: Mathys & Squire
(86) Internationale Anmeldenummer: PCT/AT2022/060250
(87) Internationale Veröffentlichungsnummer: WO 2023/283668

(56) Entgegenhaltungen:
- WO-A1-2015/100396
- US-A1- 2015 135 410

## Beschreibung

Die vorliegende Erfindung betrifft eine mobile Elektronikvorrichtung für eine Befestigung an einem menschlichen Körperteil sowie ein Elektronikset, aufweisend wenigstens zwei solcher mobilen Elektronikvorrichtungen.

Es ist bekannt, mobile Elektronikvorrichtungen für den Einsatz einer Befestigung am menschlichen Körper vorzusehen. Beispielsweise handelt es sich dabei um sogenannte Smartwatches, Fitnesstracker oder Ähnliches. Diese werden üblicherweise mit flexiblen Armbändern am Arm befestigt, um für den mobilen Einsatz dem Benutzer zur Verfügung zu stehen. Diese flexiblen Armbänder können manuell um das jeweilige Körperteil gelegt werden, um mit Verschlussmechanismen in dieser Befestigungsposition gehalten zu werden. Nachteil dieser Lösungen ist der relativ hohe Aufwand, welcher für das Umschlagen der flexiblen Armbänder und deren Befestigung notwendig ist.

Ebenfalls bekannt sind Schnappfedern, welche als passive Armbänder eingesetzt werden. Solche Schnappbänder sind lange, als Schnappfedern ausgebildete Grundkörper, welche um ein Körperteil gelegt werden können und in eine Befestigungsposition schnappen können. Der Nachteil dieser Lösungen ist die hohe Eigenspannung in diesen Schnappfedern, welche eine Kombination mit elektronischen Bauteilen erschwert und teilweise sogar unmöglich macht. Dies gilt insbesondere dahingehend, dass die Vorspannung solcher Schnappfedern ein Abdichten der Elektronikbauteile gegen Wasser und Staub sowie der Schutz vor mechanischer Belastung dieser Elektronikbauteile erschwert. Ein weiterer Nachteil dieser bekannten Schnappbänder ist die ebenfalls aufwendige Bedienung.

Die US2015/135410A1 offenbart ein Beispiel eines Schnapparmbandes.

Es ist Aufgabe der vorliegenden Erfindung, die voranstehend beschriebenen Nachteile zumindest teilweise zu beheben. Insbesondere ist es Aufgabe der vorliegenden Erfindung, in kostengünstiger und einfacher Weise eine schnelle und insbesondere einhändige Anbringmöglichkeit für eine mobile Elektronikvorrichtung an einem Körperteil zu schaffen. Vorzugsweise wird dabei gleichzeitig eine geschützte Anordnung der Elektronikbauteile gewährleistet.

Die voranstehende Aufgabe wird gelöst durch eine mobile Elektronikvorrichtung mit den Merkmalen des Anspruchs 1 sowie ein Elektronikset mit den Merkmalen des Anspruchs 22. Weitere Merkmale und Details der Erfindung ergeben sich aus den Unteransprüchen, der Beschreibung und den Zeichnungen. Dabei gelten Merkmale und Details, die im Zusammenhang mit der erfindungsgemäßen mobilen Elektronikvorrichtung beschrieben sind, selbstverständlich auch im Zusammenhang mit dem erfindungsgemäßen Elektronikset und jeweils umgekehrt, sodass bezüglich der Offenbarung zu den einzelnen Erfindungsaspekten stets wechselseitig Bezug genommen wird beziehungsweise werden kann.

Erfindungsgemäß ist eine mobile Elektronikvorrichtung dafür vorgesehen, dass sie für eine Befestigung an einem menschlichen Körperteil ausgestattet ist. Die mobile Elektronikvorrichtung weist ein Elektronikgehäuse mit darin angeordneten Elektronikbauteilen auf. An diesem Elektronikgehäuse ist auf zwei entgegengesetzt ausgerichteten Wandabschnitten jeweils ein flexibler Befestigungsarm angeordnet. Diese Befestigungsarme erstrecken sich von dem Elektronikgehäuse weg und sind zwischen einer an dem Körperteil befestigenden Befestigungsposition und einer das Körperteil freigebenden Freigabeposition flexibel verformbar. Die Befestigungsarme sind darüber hinaus in der Freigabeposition in Richtung der Befestigungsposition mit einer Vorspannkraft vorgespannt.

Ein erfindungsgemäßer Kerngedanke ist das Vorsehen einer mobilen Elektronikvorrichtung, wie dies beispielsweise bei Smartwatches, Fitnesstrackern oder Ähnlichem gegeben ist. Ein Kerngedanke ist insbesondere eine mobile Signalisierungs- oder Kommunikationseinheit, welche in der Lage ist, wie später noch erläutert wird, Lichtsignale vom Körperteil auszugeben. Hierfür können entsprechende Elektronikbauteile in Form von Leuchtmitteln, Batterievorrichtungen oder Ähnlichem vorgesehen sein.

Der erfindungsgemäße Kerngedanke einer leichten und einfachen Anbringmöglichkeit am Körperteil des Nutzers wird durch das Vorsehen von zwei separaten Befestigungsarmen gewährleistet. Diese Befestigungsarme sind jedoch im Gegensatz zu schlaffen Armbändern von Uhren vorgespannt. Die Vorspannung liegt in der Freigabeposition der Befestigungsarme vor und ist in Richtung der Befestigungsposition gerichtet. Das bedeutet, dass ein Lösen dieser Vorspannkraft in der Freigabeposition zu einer durch die Vorspannkraft geführten Bewegung der Befestigungsarme in die Befestigungsposition führt. Ähnlich den Schnappfedern, wie sie in der Einleitung erläutert worden sind, kann also eine automatische und durch die Vorspannkraft durchgeführte Bewegung aus der Freigabeposition in die Befestigungsposition, beispielsweise in schnappender Weise, erfolgen. Für das Anlegen und Befestigen an einem Körperteil kann also der Nutzer die Befestigungsarme in der vorgespannten Freigabeposition belassen und in eine gewünschte Positionierung am Körperteil des Nutzers führen. Sobald diese Positionierung, in welcher die mobile Elektronikvorrichtung befestigt werden soll, erreicht ist, löst der Nutzer, beispielsweise durch entsprechende Druckpunkte an den Befestigungsarmen, die Vorspannkraft der Freigabeposition aus, sodass die beiden Befestigungsarme aus der Freigabeposition durch die Vorspannkraft in die Befestigungsposition bewegt werden, wobei insbesondere noch eine Restvorspannkraft in der Befestigungsposition verbleibt. Diese Restvorspannkraft kann auch als verbleibende Befestigungskraft verstanden werden und dient dazu, einen Kraftschluss zwischen den Befestigungsarmen in der Befestigungsposition und dem Körperteil herzustellen.

Wie aus der voranstehenden Erläuterung ersichtlich wird, kann nun eine einhändige Befestigung am Körperteil stattfinden. Der Benutzer greift das Elektronikgehäuse mit einer Hand und führt die beiden Befestigungsarme beispielsweise auf zwei unterschiedliche Seiten seines Oberarms. Sobald diese Position der Befestigungsarme erreicht worden ist, insbesondere, wenn die Unterseite des Elektronikgehäuses die Außenseite des Oberarms als Körperteil berührt, drückt der Nutzer auf die Druckpunkte der beiden Befestigungsarme und löst damit die in der Freigabeposition gesicherte Vorspannkraft aus. Die auf diese Weise ausgelöste Vorspannkraft bewegt, beispielsweise in einer schnappenden Bewegung, die Befestigungsarme in eine gekrümmte Befestigungsposition um den Oberarm und sichert die mobile Elektronikvorrichtung somit in dieser Befestigungsposition.

Neben der einhändigen, sehr einfachen Bedienbarkeit beim Befestigen am Körperteil ist eine entsprechend ebenfalls einhändige Bedienbarkeit auch beim Abziehen vom Körperteil gegeben. Ausgehend von der Erläuterung im voranstehenden Absatz kann der Nutzer ebenfalls wieder die befestigte mobile Elektronikvorrichtung am Elektronikgehäuse greifen und einfach entgegen der ansteigenden Vorspannkraft durch Rückverformung der Befestigungsarme aus der Befestigungsposition abziehen. Insbesondere wird er dabei die Befestigungsarme durch Gegendruck gegen das Körperteil vollständig in die Freigabeposition bewegen, sodass dort die Vorspannkraft wieder durch Einrasten der Befestigungsarme in dieser Freigabeposition gesichert wird. Die vorgespannte Freigabeposition ist somit automatisch nach dem Abziehen der mobilen Elektronikvorrichtung vom Körperteil wieder vorgespannt für einen zukünftigen Befestigungsvorgang.

Neben der erleichterten Handhabbarkeit und insbesondere die einhändigen Bedienungsmöglichkeiten wird bei einer mobilen Elektronikvorrichtung der Schutz der Elektronikbauteile sehr einfach gewährleistbar. Aufgrund der Tatsache, dass eine Vorspannkraft und eine Befestigungskraft auf die Befestigungsarme wirkt, müssen diese Kräfte am Elektronikgehäuse abgestützt werden. Dies erfolgt an den entgegengesetzt ausgerichteten Wandabschnitten, insbesondere an den später noch erläuterten Aufnahmeabschnitten. Ein entscheidender Vorteil einer erfindungsgemäßen Elektronikvorrichtung liegt nun darin, dass das Elektronikgehäuse selbst, insbesondere die darin angeordneten Elektronikbauteile, nicht von den Kräften der Befestigungsarme beeinträchtigt werden. Insbesondere sind die Elektronikbauteile im Elektronikgehäuse kraftfrei beziehungsweise frei von der Vorspannkraft und/oder der Befestigungskraft der Befestigungsarme positioniert. Dies wird möglich, da die beiden Befestigungsarme voneinander getrennt sind und sich insbesondere nicht durch das Elektronikgehäuse hindurch erstrecken.

Aus der voranstehenden Erläuterung wird nun ersichtlich, dass eine funktionale Trennung zwischen der kraftbehafteten Befestigungsfunktionalität in den Befestigungsarmen und der kraftfreien Einhausung der Elektronikbauteile im Elektronikgehäuse entsteht. Dies erlaubt es, die Elektronikbauteile besser und/oder einfacher zu schützen und damit die Langlebigkeit der Funktionsweise der mobilen Elektronikvorrichtung zu erhöhen. Darüber hinaus sind gewünschte Abdichtungen gegen Staub oder Wasser deutlich einfacher zur Verfügung zu stellen, da die entsprechenden abzudichtenden Bereiche des Elektronikgehäuses unbelastet von den Befestigungskräften und den Vorspannkräften sind. Mit anderen Worten wird nun eine erleichterte Bedienung einer mobilen Elektronikvorrichtung für den Nutzer mit einer erhöhten Langlebigkeit hinsichtlich Kraftfreiheit auf die Elektronikbauteile und Dichtigkeit gegen Staub und Wasser kombinierbar. Eine kostengünstige Fertigungsmöglichkeit geht mit dieser kraftfreien Ausgestaltungsform einher.

Es ist noch darauf hinzuweisen, dass die Elektronikbauteile selbstverständlich unterschiedlichste Ausprägungen aufweisen können. So sind beliebige Sensoren, wie beispielsweise Gyroskope, Kraftsensoren, Lichtsensoren oder Ähnliches denkbar. Auch Kommunikationsmittel, beispielsweise Bluetooth-Elemente, Bluetooth Low Energy Elemente, UWB (Ultra Wide Band) Elemente, NFC-Elemente oder Mobilfunkelemente, können als Elektronikbauteile ausgewählt werden. Auch Vibrationsmotoren, akustische Aktoren, die bereits erläuterten LEDs oder Batterievorrichtungen sowie Kontrollmodule für die Kontrolle der anderen Elektronikbauteile können als Elektronikbauteile im Elektronikgehäuse angeordnet sein.

Es kann Vorteile mit sich bringen, wenn bei einer erfindungsgemäßen mobilen Elektronikvorrichtung die beiden Befestigungsarme in der Freigabeposition eine Vorspannverformung aufweisen, welche die Vorspannkraft in der Freigabeposition hält. Diese Vorspannverformung dient also insbesondere der Sicherung oder einem Einrasten der Befestigungsarme in der Freigabeposition, sodass beispielsweise durch Aktivieren von Druckpunkten auf den Befestigungsarmen diese Vorspannverformung aufgehoben werden kann, um die Vorspannkraft für eine Bewegung des Befestigungsarms aus der Freigabeposition in die Befestigungsposition freizugeben. In umgekehrter Richtung wird in der Freigabeposition der Befestigungsarm die Vorspannverformung einnehmen und auf diese Weise die Vorspannkraft absichern. Beispielsweise können die Befestigungsarme als voneinander separate Schnappfedern ausgebildet sein, welche insbesondere quer zu der Befestigungskrümmung eine zusätzliche Vorspannkrümmung aufweisen, wie sie später noch erläutert wird. Durch die Art und die Ausbildung der Vorspannverformung können auch die Druckpunkte zum Lösen dieser Vorspannverformung verschoben werden. Somit kann in der Konstruktion der Befestigungsarme vordefiniert werden, an welcher Stelle die Druckpunkte für den Befestigungsvorgang positioniert werden sollen.

Von Vorteil ist es, wenn bei einer mobilen Elektronikvorrichtung gemäß dem voranstehenden Absatz die Vorspannverformung eine Vorspannkrümmung aufweist, deren Ausrichtung sich von einer Befestigungskrümmung in der Befestigungsposition unterscheidet. Dabei ist insbesondere ein Aufnahmeabschnitt des Elektronikgehäuses vorgesehen, welcher diese Vorspannkrümmung vorgibt. Eine Vorspannverformung in einer Vorspannkrümmung ist eine besonders einfache Ausgestaltung einer solchen Vorspannverformung. Die entsprechenden Krümmungsradien und damit die zugrunde gelegten Krümmungskreise der Vorspannkrümmung und der Befestigungskrümmung sind vorzugsweise quer, insbesondere senkrecht zueinander, ausgerichtet. Die Verwendung eines Aufnahmeabschnitts im Elektronikgehäuse für eine Einspannung der Befestigungsarme kann diese Vorspannkrümmung durch eine entsprechende gekrümmte Aufnahmefläche vorgeben. Dies erlaubt es, diese Vorspannkrümmung kraftfrei in dem Aufnahmeabschnitt abzustützen, und auch bei hohen Vorspannkräften das restliche Elektronikgehäuse kraftfrei zu halten. Je stärker die Vorspannkrümmung ausgebildet ist, umso leichter schnappt der Befestigungsarm beim Lösen der Vorspannverformung aus der Freigabeposition in die Befestigungsposition. Auch verschiebt sich der Druckpunkt zum Auslösen der Vorspannkraft in Richtung des Elektronikgehäuses, sodass die Einhandbedienung noch weiter verbessert wird. Insbesondere wird es dann möglich, mit einem Zangengriff sogar gleichzeitig beide Druckpunkte von den beiden Befestigungsarmen zu drücken, sodass ein im Wesentlichen gleichzeitiges Schnappen der beiden Befestigungsarme in die Befestigungsposition erfolgt.

Von Vorteil ist es darüber hinaus, wenn bei einer erfindungsgemäßen mobilen Elektronikvorrichtung das Elektronikgehäuse an den entgegengesetzt ausgerichteten Wandabschnitten jeweils einen Aufnahmeabschnitt aufweist, in welchem ein Gegen-Aufnahmeabschnitt des jeweiligen Befestigungsarms aufgenommen ist. Ein solcher Aufnahmeabschnitt kann, wie im voranstehenden Absatz erläutert worden ist, eine gekrümmte Aufnahmefläche aufweisen, um insbesondere die Vorspannkrümmung vorzugeben. Selbstverständlich sind auf der Oberseite und der Unterseite dieses Aufnahmeabschnitts auch komplementäre, vorzugsweise ebenfalls gekrümmte Aufnahmeflächen denkbar, um eine beidseitige flächige Einspannung des Befestigungsarms ermöglichen zu können. Die Befestigung im Aufnahmeabschnitt kann durch ein Klemmen, ein Verschrauben, ein Umspritzen, ein Einrasten oder Ähnliches erfolgen. Im Vergleich zur Gesamtlänge der Befestigungsarme ist die Einspannlänge im Aufnahmeabschnitt vorzugsweise relativ kurz ausgebildet. Beispielsweise kann die Einspannlänge oder auch Aufnahmelänge der Befestigungsarme im Bereich von circa 1% bis circa 5% der Gesamtlänge der Befestigungsarme liegen
Weitere Vorteile sind erzielbar, wenn bei einer erfindungsgemäßen mobilen Elektronikvorrichtung die beiden Befestigungsarme gleichlang oder im Wesentlichen gleichlang ausgebildet sind. Dies führt dazu, dass insbesondere eine identische oder im Wesentlichen identische Ausbildung der beiden Befestigungsarme erzielt wird. Zum einen hat eine solche identische Ausgestaltung der Befestigungsarme den Vorteil, dass die mobile Elektronikvorrichtung in beiden möglichen Ausrichtungen identisch am Körperteil des Benutzers befestigt werden kann. Zum anderen ist eine gleichteilige Ausbildung beider Befestigungsarme dahingehend von Vorteil, dass die Komplexität der mobilen Elektronikvorrichtung durch die Anzahl der gleichteiligen Bauteile verkleinert wird. Neben einer leichteren und einfacheren Montage ist auf diese Weise auch eine Reduktion der Bauteilkosten erzielbar. Es ist noch darauf hinzuweisen, dass die Länge der Befestigungsarme vorzugsweise an den Durchmesser des üblicherweise für die Befestigung vorgesehenen Körperteils angepasst ist. Handelt es sich bei dem Körperteil beispielsweise um den Oberarm des Benutzers, so kann je nach Person ein minimaler Körperteilumfang und ein maximaler Körperteilumfang definiert werden. Die Befestigungsarme sind nun hinsichtlich ihrer Länge so ausgestaltet, dass sie bei maximalem Umfang des Körperteils noch eine ausreichende Umschlingung für eine feste und sichere Befestigung am Körperteil gewährleisten. Für den minimalen Umfang des Körperteils sind die Befestigungsarme vorteilhafterweise kurz genug, dass eine Überlappung der Befestigungsarme in der Befestigungsposition ein Drittel der Gesamtlänge des jeweiligen Befestigungsarms nicht überschreitet.

Weiter kann es Vorteile mit sich bringen, wenn bei einer erfindungsgemäßen mobilen Elektronikvorrichtung wenigstens einer der beiden flexiblen Befestigungsarme einen Druckpunkt aufweist, wobei die Einwirkung einer Druckkraft auf diesen Druckpunkt den Befestigungsarm in eine Löseposition bewegt, in welcher die Vorspannkraft freigegeben und der Befestigungsarm durch die freigegeben Vorspannkraft in die Befestigungsposition bewegt wird. Die Löseposition stellt demnach eine definierte Zwischenposition zwischen der Freigabeposition und der Befestigungsposition dar. Mit anderen Worten handelt es sich bei der Löseposition und die Grenze zwischen zwei Bereichen, welche eine Bewegung in die jeweils andere Richtung unterscheiden. Befindet sich der Befestigungsarm im Bereich zwischen der Freigabeposition und der Löseposition, so ist der Befestigungsarm in der Freigabeposition stabil. Dies kann zum Beispiel durch eine in die Richtung der Freigabeposition wirkende Rückstellkraft erzielt sein. Sobald eine solche Rückstellkraft durch das Einbringen der Druckkraft über wenigstens einen Druckpunkt überwunden wird, bewegt sich der Befestigungsarm in Richtung der Löseposition. Beim Überschreiten der Löseposition nimmt der Befestigungsarm eine Form ein, welche die Vorspannkraft freigibt. Beispielsweise ist in der Löseposition eine Vorspannkrümmung zumindest teilweise aufgehoben, so dass ab dieser Löseposition ohne weiter Krafteinbringung über den Druckpunkt der Befestigungsarm automatisch in die wenigstens eine Befestigungsposition bewegt wird. Diese Bewegung endet, sobald ein Widerstand erreicht wird, beispielsweise durch eine Oberfläche des Körperteils, an welchem die Elektronikvorrichtung befestigt werden soll. Mit anderen Worten schnappt der Befestigungsarm nach Überschreiten der Löseposition in die Befestigungsposition oder in Richtung der Befestigungsposition.

Auch vorteilhaft ist es, wenn bei einer erfindungsgemäßen mobilen Elektronikvorrichtung beide Befestigungsarme einen Druckpunkt, insbesondere an identischer oder im Wesentlichen identischer Position, aufweisen. Bevorzugt sind die beiden Druckpunkte symmetrisch bezüglich des Elektronikgehäuses auf die beiden Befestigungsarme verteilt. Ein dadurch erzielbarer Vorteil ist es, dass die beiden Befestigungsarme identisch ausgebildet werden können und bei symmetrischer Montage die beidseitige Befestigungsfunktion ausbilden. Die Komplexität wird hier durch eine verminderte Anzahl der Bauteile reduziert und auf diese Weise werden die Kosten minimiert. Auch ist es damit möglich in Form eines Klammergriffs mit einer menschlichen Hand einhändig die beiden Druckpunkte zu bedienen und damit den Befestigungsvorgang durchzuführen. Darüber hinaus wird durch die Symmetrie ein Anlegen an dem Körperteil in zwei unterschiedlichen Ausrichtungen möglich, wodurch die intuitive Bedienbarkeit weiter verbessert wird.

Weiter von Vorteil ist es, wenn bei einer erfindungsgemäßen mobilen Elektronikvorrichtung der wenigstens eine Druckpunkt einen Greifabstand von dem Elektronikgehäuse aufweist, welcher geringer als eine Greifbreite einer menschlichen Hand ist. Der bereits im voranstehenden Absatz beschriebene Klammergriff einer menschlichen Hand wird auf diese Weise noch leichter anwendbar. Insbesondere kann die gesamte Elektronikvorrichtung mit einem solchen Klammergriff an den Druckpunkten gehalten werden. Sobald eine Platzierung an oder um das entsprechende Körperteil erfolgt ist, kann der Nutzer den Druck mittels des Klammergriffs erhöhen, bis die eingebrachte Druckkraft eine Rückstellkraft der Befestigungsarme überschreitet. Dies leitet die Bewegung in die Löseposition ein wodurch anschließend die Schnappbewegung der Befestigungsarme die Befestigung vollendet. Hier ist gut die ergonomische und vor allem einhändige Bedienbarkeit gezeigt, welche zum Beispiel ein einhändiges Anlegen am jeweils gegenüberliegenden Oberarm des Nutzers ermöglicht.

Ebenfalls bringt es Vorteile mit sich, wenn bei einer erfindungsgemäßen mobilen Elektronikvorrichtung wenigstens ein Druckpunkt mittig oder im Wesentlichen mittig zwischen Rändern des Befestigungsarms angeordnet ist. Insbesondere bei Ausführungsformen mit einer Vorspannkrümmung kann auf diese Weise der Druckpunkt in einem tiefer liegenden Bereich zwischen den Rändern des Befestigungsarms angeordnet sein. Damit bilden die nach oben gekrümmten Ränder eine Abrutschsicherung und gleichzeitig eine Führung der Finger des Nutzers zum korrekten Druckpunkt. Auch dies verbessert die Sicherheit und Einfachheit der Nutzung weiter.

Auch vorteilhaft ist es, wenn bei einer erfindungsgemäßen mobilen Elektronikvorrichtung der wenigstens eine Druckpunkt eine Druckpunktfläche aufweist, deren Flächennormale senkrecht oder im Wesentlichen senkrecht zu einer Haupterstreckung des Befestigungsarms in der Vorspannposition ausgerichtet ist. Die Flächennormale ist dabei eine senkrechte Gerade durch die Druckpunktfläche und kann insbesondere auch als Mittelsenkrechte der Druckpunktfläche bezeichnet werden. Mit anderen Worten erfolgt bei dieser Ausführungsform die Einbringung der Druckkraft senkrecht oder im Wesentlichen senkrecht zur Druckpunktfläche und damit auch zur Haupterstreckung der Befestigungsarme. Dies erleichtert die zielgenaue Krafteinbringung und reduziert das Risiko eines Abrutschens aus dieser Position.

Darüber hinaus bringt es Vorteile mit sich, wenn bei einer erfindungsgemäßen mobilen Elektronikvorrichtung wenigstens einer der Befestigungsarme beabstandet vom Druckpunkt einen Gegen-Druckpunkt aufweist, wobei eine Einwirkung einer Gegen-Druckkraft, welche der Druckkraft entgegengerichtet ist, den Befestigungsarm von der Löseposition in die Freigabeposition bewegt und die Vorspannkraft sichert. Der Gegen-Druckpunkt ist dabei vorzugsweise weiter von dem Elektronikgehäuse entfernt als der Druckpunkt des jeweiligen Befestigungsarms. Auch sind bevorzugt auf beiden Befestigungsarmen Gegen-Druckpunkte, insbesondere symmetrisch zum Elektronikgehäuse, angeordnet. Die Gegendruckpunkte erlauben eine gezielte Führung der Befestigungsarme in die Freigabeposition und der Sicherung der Vorspannkraft. Insbesondere unterstützt die Einbringung der Gegen-Druckkraft eine Verformung des jeweiligen Befestigungsarms zur Einnahme einer Vorspannverformung, zum Beispiel in Form einer Vorspannkrümmung.

Vorteilhaft ist es ebenfalls, wenn bei einer erfindungsgemäßen mobilen Elektronikvorrichtung wenigstens einer der beiden Befestigungsarme einen Lichtleiter, insbesondere in Form einer Lichtleiterschicht, aufweist, wobei im Elektronikgehäuse wenigstens ein Leuchtmittel als Elektronikbauteil in lichtübertragendem Kontakt mit dem Lichtleiter angeordnet ist. Damit wird ein Elektronikbauteil in Form von einem oder mehreren Leuchtmitteln vorgesehen, welcher eine Lichtkommunikation nach außen erlaubt. Ein wesentlicher Vorteil ist es, dass bei dieser Ausgestaltungsform die Leuchtmittel als Elektronikbauteile in das Elektronikgehäuse integriert werden, und die Befestigungsarme damit frei von den Leuchtmitteln und damit insbesondere auch frei von Elektronikbauteilen ausgestattet werden können. So können die Leuchtmittel, zum Beispiel in Form von LEDs, auf einer Leiterplatte im Elektronikgehäuse kraftfrei, staub- und wasserdicht aufgenommen sein. Über eine flächige Kontaktierung mit einer Einkoppelfläche des Lichtleiters ist es möglich, emittiertes Licht von den Leuchtmitteln in den Lichtleiter einzubringen und entlang seiner Erstreckung entlang der Befestigungsarme zu leiten. Die Auskopplung kann dabei an separaten Bauteilen, Reflexionsflächen des Befestigungsarmes oder aber den später noch erläuterten Auskoppelflächen des Lichtleiters selbst erfolgen. Somit wird es möglich, eine Signalisierungsfunktion zur Verfügung zu stellen, mit der elektronischen Komponente vollständig innerhalb des Elektronikbauteils und einer Abstrahl- und somit einer Signalisierungsfläche, welche in die Befestigungsarme integriert wird. Diese Kombination ist nur deshalb so einfach möglich, weil die Befestigungsarme hinsichtlich ihrer Befestigungs- und Vorspannkräfte separat vom Elektronikgehäuse ausgebildet sind, sodass mechanische Belastungen der Elektronikbauteile in Form der Leuchtmittel hier im Wesentlichen vollständig vermieden werden können. Die Ausbildung des Lichtleiters in Form einer Lichtleiterschicht ist dabei besonders vorteilhaft, da sie insbesondere die Oberfläche des Befestigungsarms vollständig oder im Wesentlichen vollständig abdecken kann. Diese Lichtleiterschicht kann auf einem Grundkörper des jeweiligen Befestigungsarms befestigt sein, oder aber auch durch eine Kombination mit einer später noch erläuterten Schutzschicht, in der gewünschten Position auf den Befestigungsarmen gehalten werden. Selbstverständlich kann auch mehr als ein Leuchtmittel, insbesondere eine Vielzahl identischer Leuchtmittel in Form von LEDs, innerhalb des Elektronikgehäuses angeordnet sein. Der Lichtleiter selbst weist mindestens die gleiche Flexibilität auf, wie sie für die Verformung zwischen der Freigabeposition und der Befestigungsposition auch für die Befestigungsarme gegeben ist. Vorzugsweise sind die Leuchtmittel in dieser Ausgestaltungsform alle identisch und können insbesondere mehrere Farben gleichzeitig zur Verfügung stellen. Ein Farbwechsel ist mit solchen LEDs vorzugsweise ebenfalls möglich. Über Vertiefungen oder Durchbrüche im Grundkörper ist auch eine mechanische Befestigung des Lichtleiters am Grundkörper denkbar.

Weitere Vorteile sind erzielbar, wenn bei einer mobilen Elektronikvorrichtung der Lichtleiter Auskoppelflächen aufweist, für ein Auskoppeln des geleiteten Lichts in einer Auskoppelrichtung von den Befestigungsarmen weg. Solche Auskoppelflächen können beispielsweise Schlitze oder punktförmige Vertiefungen im Lichtleiter, insbesondere der Lichtleiterschicht, sein. Diese sind vorteilhafterweise an der Unterseite des Lichtleiters, also auf der Seite des Lichtleiters, welche die Oberseite der Befestigungsarme kontaktiert, angebracht. Die Befestigungsarme sind vorzugsweise aus einem festen Grundkörper, insbesondere aus Metall, ausgebildet. Die Auskoppelflächen können dabei einfache Vertiefungen oder Einschlitzungen im Lichtleiter sein. Alternativ oder zusätzlich zu den Einschlitzungen sind auch gedruckte Punkte oder andere Flächenformen als Auskoppelflächen auf einer der Auskoppelrichtung gegenüberliegenden Seite des Grundkörpers denkbar. Jedoch können auch zusätzliche Reflexionsflächen oder Trennelemente für die Ausbildung einzelner Lichtleitkanäle innerhalb des Lichtleiters ausgebildet sein. Die Auskoppelflächen können dabei geometrische Formen ausbilden, welche bei der Bestrahlung mit Lichtstrahlen von den Leuchtmitteln als Lichtelemente oder Lichtformen von außerhalb der mobilen Elektronikvorrichtung wahrgenommen werden können. Diese Formen für die Auskoppelflächen sind vorzugsweise ebenfalls punktförmig und/oder linienförmig ausgebildet, da dies für eine Signalisierungsfunktion von anderen Personen leichter und besser wahrnehmbar ist als eine flächige Abstrahlung des Lichts.

Weiter von Vorteil kann es sein, wenn bei einer erfindungsgemäßen mobilen Elektronikvorrichtung die Auskoppelflächen sich mit zunehmendem Abstand von dem wenigstens einen Leuchtmittel vergrößern. Da an jeder Auskoppelfläche über den Verlauf des Befestigungsarms immer ein Teil der Lichtmenge ausgekoppelt und abgestrahlt wird, nimmt die zur Verfügung stehende Lichtmenge, über den zunehmenden Abstand von den Leuchtmitteln weg, ab. Um nun vorteilhafterweise ein Angleichen der Leuchtstärke oder ein bewusstes Verteilen unterschiedlicher Leuchtstärken über den gesamten Verlauf des Lichtleiters zur Verfügung stellen zu können, wird zu Beginn, also nahe an den Leuchtmitteln, eine kleine Auskoppelfläche einen geringen Prozentsatz einer großen Lichtmenge auskoppeln, während an einem weiter entfernten Punkt von den Leuchtmitteln eine größere Auskoppelfläche eine größere prozentuale Menge an Licht von der zu diesem Punkt aber bereits reduzierten Gesamtmenge des Lichtes auskoppeln. Für andere Verteilungen der Leuchtstärke sind entsprechend andere Flächenverhältnisse zu verwenden. Bevorzugt ist die Zunahme der Auskoppelfläche korreliert mit der Abnahme der Lichtmenge, sodass eine vergleichmäßigte Abstrahlung des Lichtes von den Auskoppelflächen erzielt wird.

Vorteilhaft ist es ebenfalls, wenn bei einer erfindungsgemäßen mobilen Elektronikvorrichtung die Befestigungsarme einen Grundkörper aufweisen, welcher von einer Schutzschicht umgeben ist. Eine solche Schutzschicht kann den Befestigungsarm vorzugsweise vollständig umgeben und insbesondere auch in Aufnahmeabschnitte des Elektronikgehäuses hineinragen. Dieses Hineinragen kann in Form von Dichtlippen ausgestaltet sein, um auch hier eine möglichst staub- und wasserdichte Ausbildung zur Verfügung stellen zu können. Auch die Schutzschicht ist an die notwendige Flexibilität für eine Bewegbarkeit mit reversibler Verformung zwischen der Befestigungsposition und der Freigabeposition angepasst. So kann die Schutzschicht beispielsweise ein Silikonmaterial aufweisen, um diese Funktion zur Verfügung zu stellen. Auch kann die Schutzschicht als Schutzsocken, insbesondere als Silikonsocken ausgebildet sein, und auf diese Weise der Grundkörper in den Innenraum eines solchen Schutzsockens eingeschoben werden. Dieses Einschieben kann erleichtert werden, wenn der Silikonsocken, zum Beispiel mittel Unterdruck, in einer geöffneten Position gehalten wird. Auch ist ein vollständiges Umspritzen für das Ausbilden der Schutzschicht für den Grundkörper denkbar. Bei der Auswahl des Materials der Schutzschicht kann durch Auswahl vorteilhafter Brechungsindizes eine Erhöhung der ausgestrahlten Lichtmenge erzielt werden. Eine weitere Verbesserung der optischen Eigenschaften ist durch das Ausbilden einer Luftschicht zwischen Lichtleiter und Schutzsicht erreichbar. Ein Lichtleiter kann integral mit dieser Schutzschicht ausgebildet oder separat in diese eingebracht sein. Zusätzlich oder alternativ sind auch noch Farbschichten denkbar, welche entweder in die Schutzschicht integriert oder auf einem Grundkörper der Befestigungsarme angebracht werden. Solche Farbschichten, insbesondere aus Folien-, Pulver- oder Lackschichten, sind in der Lage, eine Personalisierung der mobilen Elektronikvorrichtung durch entsprechende Farbwahl und/oder verbesserte Reflexionseigenschaften, insbesondere durch passive Reflexionselemente, zur Verfügung zu stellen.

Von Vorteil ist es weiter, wenn bei einer erfindungsgemäßen mobilen Elektronikvorrichtung die Befestigungsarme frei oder im Wesentlichen frei von Elektronikbauteilen ausgebildet sind. Dies erlaubt es, dass die Befestigungsarme sich vollständig auf die Befestigungsfunktion konzentrieren und somit die entsprechend notwendigen Vorspannkräfte und Befestigungskräfte unabhängig von einer ansonsten notwendigen Schonung von Elektronikbauteilen erfolgen können. Die Flexibilität und auch die Bewegungen der Befestigungsarme dienen dabei der Befestigungsfunktion, ohne zu einem Verschleiß von Elektronikbauteilen der Elektronikfunktion zu führen.

Weitere Vorteile sind erzielbar, wenn bei einer erfindungsgemäßen mobilen Elektronikvorrichtung im Elektronikgehäuse Gehäuse-Lichtquellen angeordnet sind, für ein Abstrahlen von Licht durch Gehäusefenster. Diese sind insbesondere separat von den bereits erläuterten Lichtquellen für das Einkoppeln von Licht im Lichtleiter der Befestigungsarme. Die Gehäuse-Lichtquellen sind dabei vorzugsweise ebenfalls auf einer Leiterplatte angeordnet. Gehäusefenster können aus dem gleichen Material wie die restlichen Gehäuseabschnitte des Elektronikgehäuses ausgebildet sein. Jedoch sind sie vorzugsweise durch eine entsprechende Materialwahl oder durch Polieren der Oberfläche transparent ausgebildet, sodass ein möglichst hoher Anteil des von den Gehäuse-Lichtquellen erzeugten Lichts auch durch diese Gehäusefenster nach außen gelangt und dort von Personen wahrgenommen werden kann.

Weitere Vorteile bringt es mit sich, wenn bei einer erfindungsgemäßen mobilen Elektronikvorrichtung die Elektronikbauteile zumindest teilweise auf einer gemeinsamen Leiterplatte angeordnet sind, wobei insbesondere Befestigungsmittel die Leiterplatte innerhalb des Elektronikgehäuses an diesem befestigen. Eine Leiterplatte kann vorproduziert und vereinheitlicht werden, sodass die Endmontage dieser auf der Leiterplatte angeordneten Elektronikbauteile sehr einfach in dem Elektronikgehäuse stattfinden kann. Die Verwendung von Befestigungsmitteln erlaubt es, die Leiterplatte, beispielsweise über gleichmäßig verteilte Schrauben, im Elektronikgehäuse zu befestigen. So ist es auf diese Weise auch möglich, die erläuterten Leuchtmittel in Form der LEDs vorzugsweise spaltfrei an den Befestigungsarmen und dort am Lichtleiter anzulegen. Auch sind weitere Elektronikbauteile, wie beispielsweise Vibrationsmotoren, auf diese Weise spezifisch exakt mit dem Elektronikgehäuse korrelierbar, sodass im Falle eines Vibrationsmotors, dessen Vibration an die Unterseite des Elektronikgehäuses übertragen werden kann. Eine solche Leiterplatte kann aus unterschiedlichsten Materialien gefertigt sein. Für eine deutlich bessere Wärmeableitung, insbesondere, wenn ein energieintensiver und wärmeerzeugender Dauerbetrieb stattfinden soll, kann die Leiterplatte aus einem Aluminiummaterial gebildet werden.

Vorteile bringt es weiter mit sich, wenn bei einer erfindungsgemäßen mobilen Elektronikvorrichtung Fixiermittel am Gehäuse angeordnet sind, für ein Fixieren der Befestigungsarme am Elektronikgehäuse, welche insbesondere Ausrichtdome aufweisen, für ein Ausrichten der Befestigungsarme zum Elektronikgehäuse. Solche Fixiermittel können zur reversiblen oder irreversiblen Fixierung dienen. So ist grundsätzlich ein Klemmen, ein Einschnappen oder Einrasten, aber auch die Verwendung einer Verschraubung für solche Fixiermittel im Sinne der vorliegenden Erfindung denkbar. Vorzugsweise wird für jeden Befestigungsarm eine ungerade Anzahl von Fixiermitteln vorgesehen, um eine möglichst exakte Einbringung der Positionierung und auch der bereits erläuterten Vorspannkraft zu gewährleisten. Die Ausrichtdome können zum Beispiel durch entsprechende Aufnahmeöffnungen der Leiterplatte und/oder der Befestigungsarme ragen, um die Relativpositionierung der einzelnen Bauteile während der Montage zueinander sicherzustellen.

Vorteilhaft ist es weiter, wenn bei einer erfindungsgemäßen mobilen Elektronikvorrichtung das Elektronikgehäuse an seiner Unterseite eine Unterseitenkrümmung aufweist, für ein zumindest teilweise flächiges Anlegen an das Körperteil. Diese Unterseitenkrümmung ist vorzugsweise an eine Befestigungskrümmung in der Befestigungsposition angepasst und führt diese fort. Die Unterseitenkrümmung dient dazu, bei Körperteilen mit unterschiedlichen Körperteildurchmessern, einen Mittelwert auszubilden, sodass bei möglichst vielen unterschiedlichen Körperteilen ein zumindest teilweise flächiges Anlegen möglich wird. Neben einem erhöhten Komfort beim Tragen der befestigten mobilen Elektronikvorrichtung am Körperteil, dient ein flächiger Kontakt auch einer verbesserten Übertragung, wenn beispielsweise Vibrationen von einem Vibrationsmotor als Elektronikbauteil erzeugt werden sollen.

Ebenfalls Vorteile kann es mit sich bringen, wenn bei einer erfindungsgemäßen mobilen Elektronikvorrichtung die vom Elektronikgehäuse beabstandeten Arm-Enden der Befestigungsarme einen abgerundeten Rand aufweisen. Neben einer verbesserten Handhabung führt dies zu einer reduzierten Verletzungsgefahr, der ansonsten möglicherweise scharfkantige Ausgestaltung. Auch kann bei gleichbleibender Funktionalität eine Gewichtsreduktion an den Befestigungsarmen erzielt werden.

Ebenfalls Gegenstand der vorliegenden Erfindung ist ein Elektronikset, aufweisend wenigstens zwei erfindungsgemäße mobile Elektronikvorrichtungen. Damit bringt ein erfindungsgemäßes Elektronikset die gleichen Vorteile mit sich, wie sie ausführlich mit Bezug auf eine erfindungsgemäße mobile Elektronikvorrichtung erläutert worden sind. Sofern die mobilen Elektronikvorrichtungen eine definierte Ausrichtung für eine Befestigungsposition an einem linken und einem rechten Körperteil des Benutzers aufweisen, ist das Elektronikset vorzugsweise mit jeweils einer dieser Ausgestaltungsvarianten ausgebildet. Es bietet also ein Verkaufsset für die Anwendung auf beiden Körperseiten bei entsprechenden Körperteilen. Um die Ausdehnung des Elektroniksets in einer Verpackungseinheit zu reduzieren, sind die Befestigungsarme vorzugsweise in der Befestigungsposition angeordnet und miteinander verschlungen. Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung im Einzelnen beschrieben sind.

Es zeigen schematisch:
- Fig. 1: eine Ausführungsform einer mobilen Elektronikvorrichtung in Freigabeposition,
- Fig. 2: die Ausführungsform der Figur 1 während des Befestigungsvorgangs,
- Fig. 3: die Ausführungsform der Figuren 1 und 2 in der Befestigungsposition,
- Fig. 4: eine Teildarstellung einer weiteren Ausführungsform einer mobilen Elektronikvorrichtung,
- Fig. 5: die Ausführungsform der Figur 4 in der Befestigungsposition,
- Fig. 6: eine Querschnittsdarstellung zur Ausführungsform der Figur 4,
- Fig. 7: eine Querschnittsdarstellung zur Ausführungsform der Figur 5,
- Fig. 8: eine weitere Ausführungsform einer erfindungsgemäßen mobilen Elektronikvorrichtung,
- Fig. 9: ein Teilquerschnitt durch eine weitere Ausführungsform einer erfindungsgemäßen mobilen Elektronikvorrichtung,
- Fig. 10: eine Ausführungsform eines erfindungsgemäßen Elektroniksets.

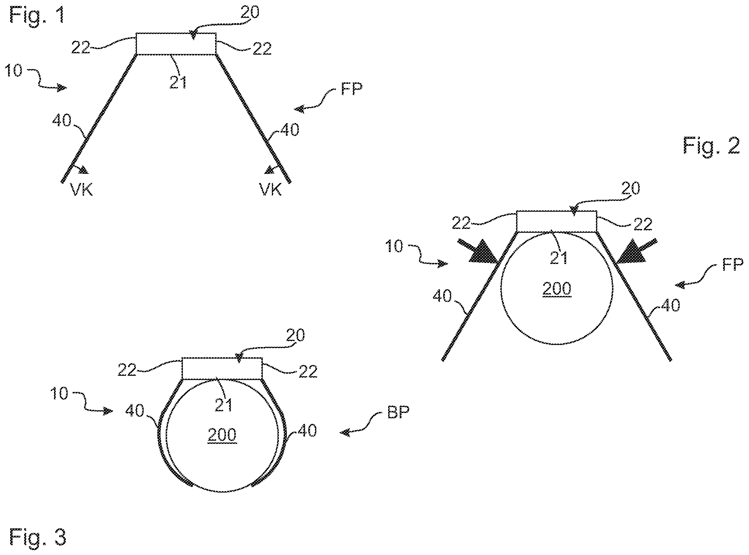

Die Figuren 1 bis 3 zeigen eine besonders einfache Ausgestaltungsform einer mobilen Elektronikvorrichtung 10 sowie der einzelnen Schritte zum Befestigen derselben an einem Körperteil 200. Das Körperteil 200 kann bei diesem Beispiel ein Oberarm eines Benutzers sein.

Um den Befestigungsvorgang zu starten, liegt dem Benutzer eine mobile Elektronikvorrichtung 10 gemäß der Figur 1 vor. In einem Elektronikgehäuse 20 ist eine Vielzahl von hier nicht näher dargestellten Elektronikbauteilen 30 angeordnet. Zwei Wandabschnitte 22 auf der linken und rechten Seite des Elektronikgehäuses 20 gehen jeweils in einen Befestigungsarm 40 über, wobei sich die beiden Befestigungsarme 40 trapezförmig nach unten erstrecken. Die Unterseite 21 des Elektronikgehäuses 20 dient dazu, an dem Körperteil 200 angelegt zu werden, wie dies die Figur 2 zeigt.

In der Figur 1 ist ebenfalls noch zu erkennen, dass in der dargestellten Freigabeposition FP der Befestigungsarme 40 jeder der beiden Befestigungsarme 40 eine Vorspannkraft VK nach innen in Richtung der Befestigungsposition BP gemäß der Figur 3 aufbringt. Durch eine später noch erläuterte Vorspannverformung VV, ist diese Vorspannkraft VK in der Freigabeposition FP gesichert, sodass ohne Aktivität durch den Benutzer diese Vorspannkraft VK nicht freigegeben werden kann.

Wird nun die Befestigung am Körperteil 200 gewünscht, greift der Benutzer die mobile Elektronikvorrichtung 10 der Figur 1 beispielswiese an den beiden Wandabschnitten 22 des Elektronikgehäuses 20 und führt die beiden Befestigungsarme 40 links und rechts an dem Körperteil 200 vorbei in die Position gemäß der Figur 2. Dabei wird insbesondere die Unterseite 21 des Elektronikgehäuses 20 auf der Oberseite des Körperteils 200 angelegt. Wie die beiden dicken Pfeile in der Figur 2 zeigen, kann nun der Benutzer hier auf Druckpunkte drücken und auf diese Weise die beiden Befestigungsarme 40 aktiv aus der Freigabeposition FP herausbewegen. Dieses Herausbewegen führt dazu, dass eine Vorspannverformung VV, wie sie später noch erläutert wird, aufgehoben wird, und die Befestigungsarme 40 durch die nun freigegebenen Vorspannkräfte VK in die Befestigungsposition BP überführt werden. Mit anderen Worten schnappen die beiden Befestigungsarme 40, angetrieben von den freigegebenen Vorspannkräften VK, nun in die Befestigungsposition BP gemäß der Figur 3. Diese zeigt die Befestigungsposition BP, wobei die beiden nun gekrümmten Befestigungsarme 40 das Körperteil 200 teilweise umschlingen und auf diese Weise das Elektronikgehäuse 20 in dieser Befestigungsposition BP am Körperteil 200 befestigen und sichern.

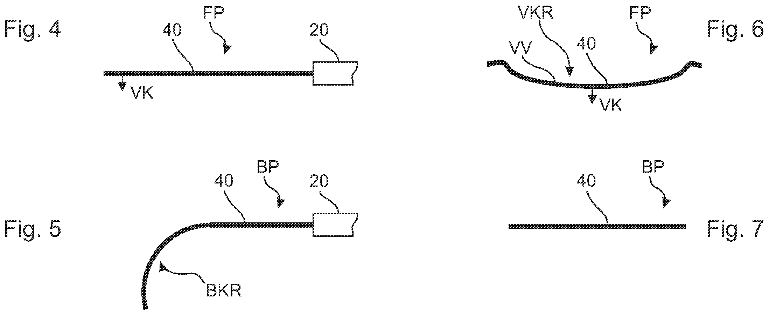

Anhand der Figuren 4, 5, 6 und 7 wird nochmals näher erläutert, wie die Vorspannkraft VK gesichert werden kann. Dies bezieht sich insbesondere auf eine Ausführungsform, wie sie mit Bezug auf die Figuren 1 bis 3 erläutert worden ist. Die Figur 4 zeigt die noch gestreckte Ausgestaltung der Befestigungsarme 40 in der Freigabeposition FP, in welcher die Vorspannkraft VK in gesicherter Weise vorliegt. Die Sicherung erfolgt durch eine Vorspannverformung VV, wie sie die Figur 6 mit einer Ansicht aus Richtung der linken Seite der Figur 4 darstellt. Diese Vorspannverformung VV weist eine Vorspannkrümmung VKR auf, welche ein Lösen des Befestigungsarmes 40 aus der ebenfalls hier dargestellten Freigabeposition FP vermeidet. Damit ist zwar eine Vorspannkraft VK gegeben, welche sich jedoch durch die Vorspannverformung VV abstützt und somit nicht freigegeben ist.

Wird nun, beispielsweise durch das Eindrücken mit einem Daumen oder einem Zeigefinger, die Vorspannverformung VV verändert, insbesondere die Vorspannkrümmung VKR aufgehoben, so führt dies dazu, dass die Sicherung der Vorspannkraft VK nicht mehr gegeben ist. Vielmehr ist diese Vorspannkraft VK freigegeben und bewegt damit durch die Einwirkung auf den Befestigungsarm 40 diesen in die Befestigungsposition BP gemäß der Figur 5 mit einer Befestigungskrümmung BKR. Die Figur 7 zeigt einen Querschnitt durch den gekrümmten Teil des Befestigungsarmes 40 der Figur 5 entlang der Längserstreckung dieses Befestigungsarms 40. Hier ist gut zu erkennen, dass in diesem gekrümmten Abschnitt des Befestigungsarms 40 nun keine Vorspannkrümmung VKR mehr vorliegt, sodass entsprechend die Befestigungsposition BP eingenommen werden konnte. Je nach Ausführungsform ist hier auch eine geringe, verbleibende Restkrümmung möglich.

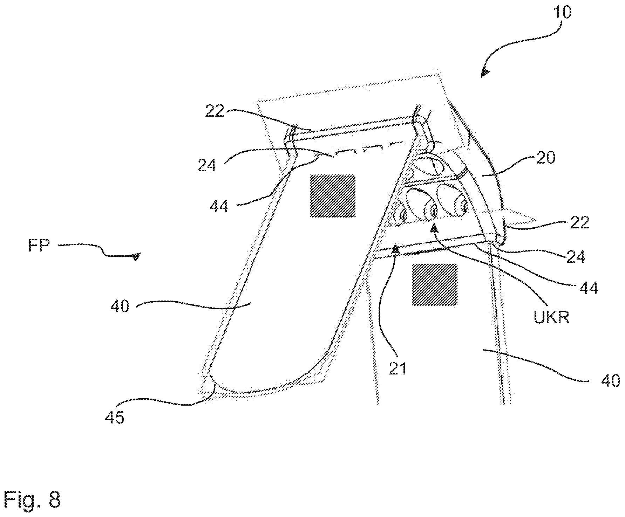

Die Figur 8 zeigt in einer Ansicht von unten eine weitere Ausführungsform einer mobilen Elektronikvorrichtung 10. Diese basiert auf der Ausgestaltung der Ausführungsform der Figuren 1 bis 7. Zusätzlich ist jedoch hier die Unterseite 21 des Elektronikgehäuses 20 mit einer Unterseitenkrümmung UKR ausgestattet, welche für ein möglichst flächiges Anlegen auf der Außenseite des Körperteils 200 zur Verfügung gestellt ist. Sie führt insbesondere die Befestigungskrümmung BKR zumindest teilweise fort, um auch den Komfort beim Tragen an dem Körperteil 200 zu erhöhen. Mit zwei Quadraten sind hier nahe bei den Wandabschnitten 22 gelegene Druckabschnitte dargestellt, welche mit einem Zangengriff mit Daumen und Zeigefinger des Benutzers gegriffen und gedrückt werden können, um die Vorspannverformung, wie sie mit Bezug auf die Figuren 4 bis 7 erläutert worden ist, aufzuheben. Die Darstellungsform der Figur 8 zeigt die Befestigungsarme 40 in der Freigabeposition FP.

Ebenfalls gut in Figur 8 zu erkennen, ist, dass die oberen Enden des Befestigungsarms 40 jeweils einen Gegen-Aufnahmeabschnitt 44 aufweisen, welcher in einen Aufnahmeabschnitt 24 des Elektronikgehäuses 20 eingesteckt ist. Dieses Einstecken erfolgt in gekrümmter Weise, sodass die dort vorhandenen Auflageflächen, insbesondere in komplementärer Weise, die Vorspannverformung VV für den Befestigungsarm 40 vorgeben. Ebenfalls kann der Figur 8 eine Variante entnommen werden, bei welcher am Arm-Ende 45 des Befestigungsarmes 40 eine Rundung vorgesehen ist, um die Verletzungsgefahr zu reduzieren und das Gewicht der Befestigungsarme 40 zu minimieren.

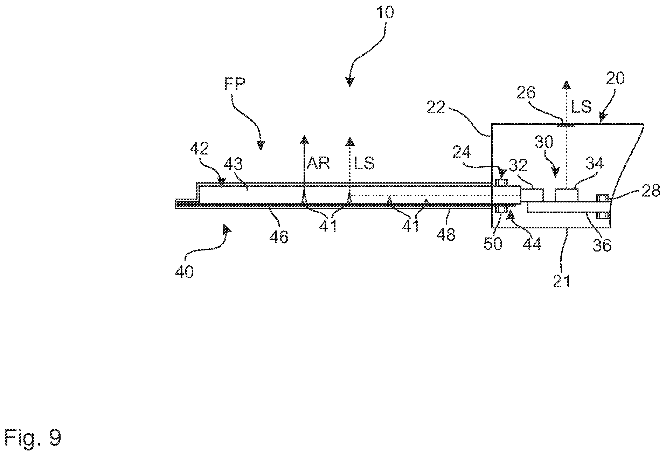

Die Figur 9 zeigt im Querschnitt ein mögliches Innenleben einer mobilen Elektronikvorrichtung 10. Hier ist der Befestigungsarm 40 unter anderem mit einer Schutzschicht 48 umgeben, welche als sockenartiger Silikonschutz vorgesehen ist. Diese Schutzschicht 48 ragt in einen Aufnahmeabschnitt 44 des Elektronikgehäuses 20 mit ein und dient dort einer staub- und wasserdichten Abdichtung. Innerhalb der Schutzschicht 48 ist neben einem metallischen Grundkörper 46 ein Lichtleiter 42 als Lichtleiterschicht 43 angeordnet. Diese Lichtleiterschicht 43 ist transparent und kann Licht von einem Leuchtmittel 32 aufnehmen.

Das Leuchtmittel 32 ist dabei auf einer Leiterplatte 36 innerhalb des Elektronikgehäuses 20 angeordnet. Diese Leiterplatte 36 ist mithilfe von Befestigungsmitteln 28, hier einer Verschraubung, am Elektronikgehäuse 20 befestigt. Diese Verschraubung dient insbesondere auch dazu, eine spaltfreie Kontaktierung mit dem Lichtleiter 42 zu gewährleisten, sodass Lichtstrahlen LS von dem Leuchtmittel 32 in den Lichtleiter 42 eingekoppelt werden können. Dies erfolgt im Bereich des Gegen-Aufnahmeabschnitts 44, welcher mithilfe von Fixiermitteln 50 eine fixierende Anordnung des Befestigungsarms 40 in dieser Position erlaubt.

Für das Auskoppeln von Lichtstrahlen LS sind hier im Querschnitt dargestellte unterseitige Schlitze als Auskoppelflächen 41 im Lichtleiter 42 vorgesehen. Die Auskoppelrichtung AR ist dabei im Wesentlichen senkrecht zum Grundkörper 46 ausgerichtet, sodass in der gleichen Richtung die Lichtstrahlen LS ausgekoppelt und von außen wahrnehmbar sind.

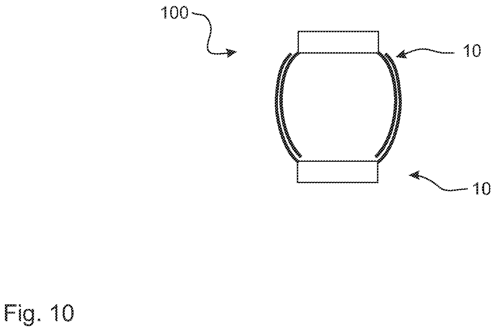

Zusätzlich ist in der Figur 9 noch eine Variante dargestellt, bei welcher auf der Leiterplatte 36 eine Gehäuse-Lichtquelle 34 angeordnet ist. Auch hierbei kann es sich um LEDs handeln, welche einen Lichtstrahl LS abgeben, welcher durch ein Gehäusefenster 26 von außen wahrnehmbar ist. Auf diese Weise können ergänzende oder alternativ zusätzliche Lichtfunktionen als Signalisierungsfunktion erzeugt werden. Die Figur 10 zeigt ein Elektronikset 100, welches zwei mobile Elektronikvorrichtungen 10 aufweist. Dabei kann es sich insbesondere um Elektronikvorrichtungen 10 gemäß der Figuren 1 bis 3 handeln. Wird eine Unterscheidung für die linke und die rechte Seite eines Benutzers gemacht, so besteht dieses Elektronikset 100 aus einer linken und einer rechten Ausführungsform der mobilen Elektronikvorrichtung 10. Bei dieser Darstellung ist auch gut zu erkennen, dass sich beide Befestigungsarme 40 beider Elektronikvorrichtungen 10 in der Befestigungsposition BP befinden und einander umschlingen, sodass der Platzbedarf für eine Verpackungseinheit für dieses Elektronikset reduziert ist.

Die voranstehende Erläuterung der Ausführungsformen beschreibt die vorliegende Erfindung ausschließlich im Rahmen von Beispielen. Selbstverständlich können einzelne Merkmale der Ausführungsformen, sofern technisch sinnvoll, frei miteinander kombiniert werden, ohne den Rahmen der vorliegenden Erfindung, die ausschließlich durch die Ansprüche definiert ist, zu verlassen.

### Bezugszeichenliste

- 10: Mobile Elektronikvorrichtung
- 20: Elektronikgehäuse
- 22: Wandabschnitt
- 21: Unterseite
- 24: Aufnahmeabschnitt
- 26: Gehäusefenster
- 28: Befestigungsmittel
- 30: Elektronikbauteil
- 32: Leuchtmittel
- 34: Gehäuse-Lichtquelle
- 36: Leiterplatte
- 40: Befestigungsarm
- 41: Auskoppelfläche
- 42: Lichtleiter
- 43: Lichtleiterschicht
- 44: Gegen-Aufnahmeabschnitt
- 45: Arm-Ende
- 46: Grundkörper
- 48: Schutzschicht
- 50: Fixiermittel

- 100: Elektronikset
- 200: Körperteil

- BP: Befestigungsposition
- BKR: Befestigungskrümmung
- FP: Freigabeposition
- VK: Vorspannkraft
- VV: Vorspannverformung
- VKR: Vorspannkrümmung
- UKR: Unterseitenkrümmung

- AR: Auskoppelrichtung
- LS: Lichtstrahl

## Patentansprüche

1. Mobile Elektronikvorrichtung (10) für eine Befestigung an einem menschlichen Körperteil (200), aufweisend ein Elektronikgehäuse (20) mit darin angeordneten Elektronikbauteilen (30), wobei an dem Elektronikgehäuse (20) auf zwei entgegengesetzt ausgerichteten Wandabschnitten (22) jeweils ein flexibler Befestigungsarm (40) angeordnet ist, welche sich von dem Elektronikgehäuse (20) weg erstrecken und zwischen einer an dem Körperteil (200) befestigenden Befestigungsposition (BP) und einer das Körperteil (200) freigebenden Freigabeposition (FP) flexibel verformbar sind, die Elektronikvorrichtung ist **dadurch gekennzeichnet dass**, die Befestigungsarme (40) in der Freigabeposition (FP) in Richtung der Befestigungsposition (BP) mit einer Vorspannkraft (VK) vorgespannt sind.

2. Mobile Elektronikvorrichtung (10) nach Anspruch 1, **dadurch gekennzeichnet, dass** die beiden Befestigungsarme (40) in der Freigabeposition (FP) eine Vorspannverformung (VV) aufweisen, welche die Vorspannkraft (VK) in der Freigabeposition (FP) hält.

3. Mobile Elektronikvorrichtung (10) nach Anspruch 2, **dadurch gekennzeichnet, dass** die Vorspannverformung (VV) eine Vorspannkrümmung (VKR) aufweist, deren Ausrichtung sich von einer Befestigungskrümmung (BKR) in der Befestigungsposition (BP) unterscheidet, wobei insbesondere ein Aufnahmeabschnitt (24) des Elektronikgehäuses (20) diese Vorspannkrümmung (VKR) vorgibt.

4. Mobile Elektronikvorrichtung (10) nach einem der vorangegangenen Ansprüche, **dadurch gekennzeichnet, dass** das Elektronikgehäuse (20) an den entgegengesetzt ausgerichteten Wandabschnitten (22) jeweils einen Aufnahmeabschnitt (24) aufweist, in welchem ein Gegen-Aufnahmeabschnitt (44) des jeweiligen Befestigungsarms (40) aufgenommen ist, und/oder die beiden Befestigungsarme (40) gleich lang oder im Wesentlichen gleich lang ausgebildet sind.

5. Mobile Elektronikvorrichtung (10) nach einem der vorangegangenen Ansprüche, **dadurch gekennzeichnet, dass** wenigstens einer der beiden flexiblen Befestigungsarme (40) einen Druckpunkt aufweist, wobei die Einwirkung einer Druckkraft auf diesen Druckpunkt den Befestigungsarm (40) in eine Löseposition bewegt, in welcher die Vorspannkraft (VK) freigegeben und der Befestigungsarm (40) durch die freigegeben Vorspannkraft (VK) in die Befestigungsposition (BP) bewegt wird.

6. Mobile Elektronikvorrichtung (10) nach Anspruch 5, **dadurch gekennzeichnet, dass** beide Befestigungsarme (40) einen Druckpunkt, insbesondere an identischer oder im Wesentlichen identischer Position, aufweisen.

7. Mobile Elektronikvorrichtung (10) nach einem der Ansprüche 5 oder 6, **dadurch gekennzeichnet, dass** der wenigstens eine Druckpunkt einen Greifabstand von dem Elektronikgehäuse (20) aufweist, welcher geringer als eine Greifbreite einer menschlichen Hand ist.

8. Mobile Elektronikvorrichtung (10) nach einem der Ansprüche 5 bis 7, **dadurch gekennzeichnet, dass** der wenigstens eine Druckpunkt mittig oder im Wesentlichen mittig zwischen Rändern des Befestigungsarms (40) angeordnet ist.

9. Mobile Elektronikvorrichtung (10) nach einem der Ansprüche 5 bis 8, **dadurch gekennzeichnet, dass** der wenigstens eine Druckpunkt eine Druckpunktfläche aufweist, deren Flächennormale senkrecht oder im Wesentlichen senkrecht zu einer Haupterstreckung des Befestigungsarms (40) in der Freigabeposition (FP) ausgerichtet ist.

10. Mobile Elektronikvorrichtung (10) nach einem der Ansprüche 5 bis 9, **dadurch gekennzeichnet, dass** wenigstens einer der Befestigungsarme (40) beabstandet vom Druckpunkt einen Gegen-Druckpunkt aufweist, wobei eine Einwirkung einer Gegen-Druckkraft, welche der Druckkraft entgegengerichtet ist, den Befestigungsarm (40) von der Löseposition in die Freigabeposition (FP) bewegt und die Vorspannkraft (VK) sichert.

11. Mobile Elektronikvorrichtung (10) nach einem der vorangegangenen Ansprüche, **dadurch gekennzeichnet, dass** wenigstens einer der beiden Befestigungsarme (40) einen Lichtleiter (42), insbesondere in Form einer Lichtleiterschicht (43), aufweist, wobei im Elektronikgehäuse (20) wenigstens ein Leuchtmittel (32) als Elektronikbauteil (30) in lichtübertragendem Kontakt mit dem Lichtleiter (42) angeordnet ist,
wobei bevorzugt der Lichtleiter (42) Auskoppelflächen (41) aufweist für ein Auskoppeln des geleiteten Lichts in einer Auskoppelrichtung (AR) von den Befestigungsarmen (40) weg, wobei ferner bevorzugt die Auskoppelflächen (41) sich mit zunehmendem Abstand von dem wenigstens einen Leuchtmittel (32) verändern, insbesondere vergrößern.

12. Mobile Elektronikvorrichtung (10) nach einem der vorangegangenen Ansprüche, **dadurch gekennzeichnet, dass** die Befestigungsarme (40) einen Grundkörper (46) aufweisen, welcher von einer Schutzschicht (48) umgeben ist, und/oder
die Befestigungsarme (40) frei oder im Wesentlichen frei von Elektronikbauteilen (30) ausgebildet sind, und/oder
die vom Elektronikgehäuse (20) beabstandeten Arm-Enden (45) der Befestigungsarme (40) einen abgerundeten Rand aufweisen.

13. Mobile Elektronikvorrichtung (10) nach einem der vorangegangenen Ansprüche, **dadurch gekennzeichnet, dass** im Elektronikgehäuse (20) Gehäuse-Lichtquellen (34) angeordnet sind für ein Abstrahlen von Licht durch Gehäusefenster (26), und/oder
das Elektronikgehäuse (20) an seiner Unterseite (21) eine Unterseitenkrümmung (UKR) aufweist für ein zumindest teilweise flächiges Anlegen an das Körperteil (200), und/oder
Fixiermittel (50) am Elektronikgehäuse (20) angeordnet sind für ein Fixieren der Befestigungsarme (40) am Elektronikgehäuse (20), welche insbesondere Ausrichtdome aufweisen für ein Ausrichten der Befestigungsarme (40) zum Elektronikgehäuse (20).

14. Mobile Elektronikvorrichtung (10) nach einem der vorangegangenen Ansprüche, **dadurch gekennzeichnet, dass** die Elektronikbauteile (30) zumindest teilweise auf einer gemeinsamen Leiterplatte (36) angeordnet sind, wobei insbesondere Befestigungsmittel (28) die Leiterplatte (36) innerhalb des Elektronikgehäuses (20) an diesem befestigen.

15. Elektronikset (100), aufweisend wenigstens zwei mobile Elektronikvorrichtungen (10) mit den Merkmalen eines der Ansprüche 1 bis 14.

## Claims

1. Mobile electronic device (10) for attachment to a human body part (200), comprising an electronic housing (20) with electronic components (30) arranged therein, wherein a flexible fastening arm (40) is arranged on the electronic housing (20) on each of two oppositely oriented wall sections (22), which extend away from the electronic housing (20) and are flexibly deformable between a fastening position (BP) fastening to the body part (200) and a release position (FP) releasing the body part (200), **characterised in that** the fastening arms (40) are prestressed in the release position (FP) in the direction of the fastening position (BP) with a prestressing force (VK).

2. Mobile electronic device (10) according to claim 1, **characterised in that** the two fastening arms (40) have a prestressing deformation (VV) in the release position (FP), which holds the prestressing force (VK) in the release position (FP).

3. Mobile electronic device (10) according to claim 2, **characterised in that** the prestressing deformation (VV) has a prestressing curvature (VKR), the orientation of which differs from a fastening curvature (BKR) in the fastening position (BP), wherein in particular a receiving section (24) of the electronic housing (20) predetermines this prestressing curvature (VKR).

4. Mobile electronic device (10) according to one of the preceding claims, **characterised in that** the electronic housing (20) has a receiving section (24) on each of the oppositely aligned wall sections (22), in which a counter-receiving section (44) of the respective fastening arm (40) is received, and/or the two fastening arms (40) are of the same length or substantially the same length.

5. Mobile electronic device (10) according to one of the preceding claims, **characterised in that** at least one of the two flexible fastening arms (40) has a pressure point, wherein the action of a pressure force on this pressure point moves the fastening arm (40) into a release position in which the prestressing force (VK) is released and the fastening arm (40) is moved into the fastening position (BP) by the released prestressing force (VK).

6. Mobile electronic device (10) according to claim 5, **characterised in that** both fastening arms (40) have a pressure point, in particular at an identical or substantially identical position.

7. Mobile electronic device (10) according to one of claims 5 or 6, **characterised in that** the at least one pressure point has a gripping distance from the electronic housing (20) which is less than a gripping width of a human hand.

8. Mobile electronic device (10) according to any one of claims 5 to 7, **characterised in that** the at least one pressure point is arranged centrally or substantially centrally between edges of the fastening arm (40).

9. Mobile electronic device (10) according to one of claims 5 to 8, **characterised in that** the at least one pressure point has a pressure point surface, the surface normal of which is aligned perpendicularly or substantially perpendicularly to a main extension of the fastening arm (40) in the release position (FP).

10. Mobile electronic device (10) according to one of claims 5 to 9, **characterised in that** at least one of the fastening arms (40) has a counter-pressure point at a distance from the pressure point, wherein an action of a counter-pressure force, which is directed in the opposite direction to the pressure force, moves the fastening arm (40) from the release position into the release position (FP) and secures the prestressing force (VK).

11. Mobile electronic device (10) according to one of the preceding claims, **characterised in that** at least one of the two fastening arms (40) has a light guide (42), in particular in the form of a light guide layer (43), wherein at least one illuminant (32) is arranged in the electronic housing (20) as an electronic component (30) in light-transmitting contact with the light guide (42), wherein the light guide (42) preferably has decoupling surfaces (41) for decoupling the guided light in a decoupling direction (AR) away from the fastening arms (40), wherein furthermore the decoupling surfaces (41) preferably change, in particular increase, with increasing distance from the at least one illuminant (32).

12. Mobile electronic device (10) according to one of the preceding claims, **characterised in that** the fastening arms (40) have a base body (46) which is surrounded by a protective layer (48), and/or the fastening arms (40) are formed free or substantially free of electronic components (30), and/or the arm ends (45) of the fastening arms (40) spaced from the electronic housing (20) have a rounded edge.

13. Mobile electronic device (10) according to one of the preceding claims, **characterised in that** housing light sources (34) are arranged in the electronic housing (20) for emitting light through housing windows (26), and/or the electronic housing (20) has an underside curvature (UKR) on its underside (21) for at least partial flat contact with the body part (200), and/or fixing means (50) are arranged on the electronic housing (20) for fixing the fastening arms (40) to the electronic housing (20), which in particular have alignment domes for aligning the fastening arms (40) with the electronic housing (20).

14. Mobile electronic device (10) according to one of the preceding claims, **characterised in that** the electronic components (30) are arranged at least partially on a common printed circuit board (36), wherein in particular fastening means (28) fasten the printed circuit board (36) within the electronic housing (20) to the latter.

15. An electronic set (100) comprising at least two mobile electronic devices (10) having the features of any one of claims 1 to 14.

## Revendications

1. Dispositif électronique mobile (10) destiné à être fixé sur une partie du corps humain (200), présentant un boîtier électronique (20) avec des composants électroniques (30) disposés à l'intérieur, dans lequel un bras de fixation flexible (40) est disposé sur deux sections de paroi (22) orientées en sens inverse sur le boîtier électronique (20), qui s'étendent à partir du boîtier électronique (20) et sont déformables de manière flexible entre une position de fixation (BP) fixant la partie de corps (200) et une position de libération (FP) libérant la partie de corps (200), **caractérisé en ce que** les bras de fixation (40) sont précontraints dans la position de libération (FP) en direction de la position de fixation (BP) avec une force de précontrainte (VK).

2. Dispositif électronique mobile (10) selon la revendication 1, **caractérisé en ce que** les deux bras de fixation (40) présentent, en position de libération (FP), une déformation de précontrainte (VV) qui maintient la force de précontrainte (VK) en position de libération (FP).

3. Dispositif électronique mobile (10) selon la revendication 2, **caractérisé en ce que** la déformation de précontrainte (VV) présente une courbure de précontrainte (VKR) dont l'orientation est différente d'une courbure de fixation (BKR) en position de fixation (BP), **caractérisé en ce qu'**une partie de réception (24) du boîtier électronique (20) impose cette courbure de précontrainte (VKR).

4. Dispositif électronique mobile (10) selon l'une des revendications précédentes, **caractérisé en ce que** le boîtier électronique (20) présente, sur les sections de paroi (22) orientées en sens inverse, respectivement une section de réception (24) dans laquelle est reçue une section de réception opposée (44) du bras de fixation (40) respectif, et/ou les deux bras de fixation (40) sont de même longueur ou sensiblement de même longueur.

5. Dispositif électronique mobile (10) selon l'une quelconque des revendications précédentes, **caractérisé en ce qu'**au moins l'un des deux bras de fixation flexibles (40) comporte un point de pression, l'application d'une force de pression sur ce point de pression déplaçant le bras de fixation (40) vers une position de libération dans laquelle la force de précontrainte (VK) est libérée et le bras de fixation (40) est déplacé vers la position de fixation (BP) par la force de précontrainte libérée (VK).

6. Dispositif électronique mobile (10) selon la revendication 5, **caractérisé en ce que** les deux bras de fixation (40) présentent un point de pression, notamment à une position identique ou sensiblement identique.

7. Dispositif électronique mobile (10) selon l'une des revendications 5 ou 6, **caractérisé en ce que** ledit au moins un point de pression présente une distance de préhension par rapport au boîtier électronique (20) qui est inférieure à une largeur de préhension d'une main humaine.

8. Dispositif électronique mobile (10) selon l'une des revendications 5 à 7, **caractérisé en ce que** ledit au moins un point de pression est situé au centre ou sensiblement au centre entre des bords du bras de fixation (40).

9. Dispositif électronique mobile (10) selon l'une des revendications 5 à 8, **caractérisé en ce que** ledit au moins un point de pression présente une surface de point de pression dont la normale à la surface est orientée perpendiculairement ou sensiblement perpendiculairement à une extension principale du bras de fixation (40) dans la position de libération (FP).

10. Dispositif électronique mobile (10) selon l'une quelconque des revendications 5 à 9, **caractérisé en ce qu'**au moins l'un des bras de fixation (40) comporte, à distance du point de pression, un point de pression antagoniste, une action d'une force de pression antagoniste, opposée à la force de pression, déplaçant le bras de fixation (40) de la position de desserrage à la position de libération (FP) et assurant la force de précontrainte (VK).

11. Dispositif électronique mobile (10) selon l'une des revendications précédentes, **caractérisé en ce qu'**au moins l'un des deux bras de fixation (40) présente un guide de lumière (42), en particulier sous la forme d'une couche de guide de lumière (43), au moins un moyen d'éclairage (32) étant disposé dans le boîtier électronique (20) comme composant électronique (30) en contact de transmission de lumière avec le guide de lumière (42), dans lequel de préférence le guide de lumière (42) présente des surfaces de découplage (41) pour un découplage de la lumière guidée dans une direction de découplage (AR) en s'éloignant des bras de fixation (40), dans lequel en particulier les surfaces de découplage (41) se modifient, en particulier s'agrandissent, avec une distance croissante par rapport à l'au moins un moyen d'éclairage (32).

12. Dispositif électronique mobile (10) selon l'une des revendications précédentes, **caractérisé en ce que** les bras de fixation (40) présentent un corps de base (46) qui est entouré d'une couche de protection (48), et/ou les bras de fixation (40) sont réalisés sans ou sensiblement sans composants électroniques (30), et/ou les extrémités de bras (45) des bras de fixation (40), situées à distance du boîtier électronique (20), présentent un bord arrondi.

13. Dispositif électronique mobile (10) selon l'une des revendications précédentes, **caractérisé en ce que** des sources lumineuses de boîtier (34) sont disposées dans le boîtier électronique (20) pour un rayonnement de lumière à travers des fenêtres de boîtier (26), et/ou le boîtier électronique (20) présente sur sa face inférieure (21) une courbure de face inférieure (UKR) pour une application au moins partiellement plane sur la partie du corps (200), et/ou des moyens de fixation (50) sont disposés sur le boîtier électronique (20) pour une fixation des bras de fixation (40) sur le boîtier électronique (20), lesquels présentent en particulier des dômes d'alignement pour un alignement des bras de fixation (40) par rapport au boîtier électronique (20).

14. Dispositif électronique mobile (10) selon l'une quelconque des revendications précédentes, **caractérisé en ce que** les composants électroniques (30) sont disposés au moins partiellement sur une carte de circuit imprimé commune (36), dans lequel en particulier des moyens de fixation (28) fixent la carte de circuit imprimé (36) à l'intérieur du boîtier électronique (20).

15. Ensemble électronique (100) comprenant au moins deux dispositifs électroniques mobiles (10) ayant les caractéristiques de l'une des revendications 1 à 14.
